# EUROPEAN PATENT APPLICATION

(11) **EP 2 299 270 A1**
(43) Date of publication of application: **23.03.2011**
(21) Application number: 09797785.4
(22) Date of filing: 19.06.2009
(51) Int. Cl.: G01N 33/531, G01N 33/543, G01N 33/553

(54) **METHOD FOR STABILIZING MICROPARTICLES HAVING REACTIVE SUBSTANCE BOUND THERETO, AND REAGENT CONTAINING THE MICROPARTICLES**

(30) Priority: 14.07.2008 JP 2008182728
(71) Applicant: Alfresa Pharma Corporation, Chuo-ku Osaka-shi Osaka 540-8575 (JP)
(72) Inventor: TANAKA, Mutsumi, Ibaraki-shi Osaka 567-0806 (JP)
(74) Representative: Arth, Hans-Lothar
(86) International application number: PCT/JP2009/061214
(87) International publication number: WO 2010/007857

(57) **Abstract**

It is an object of the present invention to provide a method for maintaining the reactivity of microparticles having a reactive substance bound thereto in a dispersion liquid of the microparticles over a long period of time by stabilizing the microparticles in the dispersion liquid. The invention provides a method for stabilizing microparticles having a reactive substance bound thereto, which includes the step of allowing a sulfur-containing amino acid or a derivative thereof to coexist in a dispersion liquid of the microparticles. As for the sulfur-containing amino acid, cysteine, cystine, and methionine are preferable. The method of the invention is particularly useful when a precipitation preventing substance, such as a polyanion, dextran, or the like, is allowed to coexist in the dispersion liquid.

## Description

### Technical Field

The present invention relates to a method for stabilizing microparticles having a reactive substance, such as an antibody or an antigen, bound thereto (to which is, hereinafter, referred also as reactive substance-bound microparticles) and an immunoassay reagent that uses such reactive substance-bound microparticles. In particular, the invention relates to a method for stabilizing reactive substance-bound microparticles for use in an immunological measurement that takes advantage of an antigen-antibody reaction mainly in the field of clinical examination, and an immunoassay reagent that uses such reactive substance-bound microparticles.

### Background Art

Recent years, attempts at automation and measurement time reduction have been seen in clinical examinations and various other examinations. For example, methods for measuring a small amount of a substance present in a biological sample using immunological reaction are widely used. Examples of such immunoassay methods include RIA, EIA, immunonephelometry, latex agglutination, colloidal gold agglutination, and immunochromatography. Among them, methods by using microparticles to which a reactive substance is bound (reactive substance-bound microparticles), such as latex agglutination, colloidal gold agglutination, and the like, do not require separation or washing operations on the reaction fluid and are thus suitable for automating the measurement and shortening the measurement time.

However, the latex agglutination and colloidal gold agglutination are problematic in that it is difficult to store a reagent over a long period of time without deterioration of measurement accuracy, since reactive substance-bound microparticles are unstable in a dispersion liquid and the reactivity cannot be maintained over a long period of time due to precipitation, autoagglutination, or the like.

Patent Document 1 discloses a method for preventing the precipitation of reactive substance-bound microparticles, including the step of allowing at least one selected from the group consisting of a polyanion and a salt thereof, dextran, cyclodextrin, polyethylene glycol, and glycerol to coexist in a dispersion liquid of the reactive substance-bound microparticles. This method aims to stabilize reactive substance-bound microparticles by preventing the precipitation of the reactive substance-bound microparticles in a reagent solution. However, this method is problematic in that once a reagent that contains the reactive substance-bound microparticles is stored over a long period of time, the reactive substance-bound microparticles may undergo autoagglutination and the reactivity may be deteriorated.

Patent Document 2 discloses the addition of a metal ion and a chelating material as a stabilizer, and Patent Document 3 discloses the addition of a calcium ion, a magnesium ion, dextran sulfate, cholic acid, deoxycholic acid, xanthurenic acid, or a salt thereof. However, problems such as autoagglutination and deterioration of reactivity still remain.

Patent Document 4 discloses a method for preparing gold nanoparticles having a cationic surface, characterized by reducing chloroauric acid in the presence of a compound having a thiol group and an amino group (Claim 1). Patent Document 5 discloses a method for synthesizing tiopronin monolayer-protected gold nanoparticles, which are metallic nanoparticles coated with a mixed monolayer (Example 3). Patent Document 6 discloses an immunoreactive agglomerating agent in which a peptide containing at least one cysteine is bound to gold particles via a sulfur atom present in the cysteine (Claim 1). However, these documents only disclose methods for preparing gold particles on the surface of which a substance is bound, for example, in order to enhance reactivity, but do not disclose a method for stabilizing gold particles.

### Prior Art Document

### Patent Document

Patent Document 1: Japanese Laid-Open Patent Publication No. 2007-114129
Patent Document 2: Japanese Laid-Open Patent Publication No. 2000-146967
Patent Document 3: Japanese Laid-Open Patent Publication No. H11-101799
Patent Document 4: Japanese Laid-Open Patent Publication No. 2005-287507
Patent Document 5: Japanese National Publication No. 2005-534810
Patent Document 6: Japanese Laid-Open Patent Publication No. 2006-317401

### Summary of the Invention

### Problems To Be Solved By the Invention

Therefore, in order to store a reagent over a long period of time without deterioration of measurement accuracy, there are demands for a technique for stabilizing reactive substance-bound microparticles in a dispersion liquid and maintaining the reactivity over a long period of time.

Accordingly, it is an object of the present invention to provide a method for stabilizing reactive substance-bound microparticles in a dispersion liquid thereof. Also, it is an object of the invention to provide a reagent that contains reactive substance-bound microparticles with extended reactivity and that can be stored over a long period of time without deterioration of measurement accuracy.

### Means for Solving the Problems

The inventors have conducted extensive research to achieve the aforementioned objects, and found that by allowing a sulfur-containing amino acid or a derivative thereof to coexist in a dispersion liquid of microparticles having a reactive substance bound thereto, the microparticles having a reactive substance bound thereto can be stabilized and the reactivity of the microparticles having a reactive substance bound thereto can be maintained over a long period of time, and thus the inventors arrived at the present invention.

The invention provides a method for stabilizing microparticles having a reactive substance bound thereto, the method including the step of allowing a sulfur-containing amino acid or a derivative thereof to coexist in a dispersion liquid of the microparticles.

In one embodiment, the sulfur-containing amino acid is at least one selected from the group consisting of cysteine, cystine, and methionine.

In another embodiment, the sulfur-containing amino acid or the derivative thereof is contained in the dispersion liquid in a proportion of 0.001 to 2.0 weight percent (wt%).

In another embodiment, the method includes the step of allowing a precipitation preventing substance to further coexist in the dispersion liquid.

In another embodiment, the precipitation preventing substance is at least one selected from the group consisting of polyanions and salts thereof, dextran, cyclodextrin, polyethylene glycol, and glycerol.

In another embodiment, the polyanion is dextran sulfate or heparin.

In another embodiment, the microparticles having a reactive substance bound thereto are colloidal gold particles having an antibody or an antigen bound thereto.

The reagent of the invention contains microparticles having a reactive substance bound thereto and a sulfur-containing amino acid or a derivative thereof thereby stabilizing the microparticles.

In one embodiment, the sulfur-containing amino acid is at least one selected from the group consisting of cysteine, cystine, and methionine.

In another embodiment, the sulfur-containing amino acid or the derivative thereof is contained in the reagent in a proportion of 0.001 to 2.0 wt%.

In another embodiment, the reagent further contains a precipitation preventing substance.

In another embodiment, the precipitation preventing substance is at least one selected from the group consisting of polyanions and salts thereof, dextran, cyclodextrin, polyethylene glycol, and glycerol.

In another embodiment, the polyanion is dextran sulfate or heparin.

In another embodiment, the microparticles having a reactive substance bound thereto are colloidal gold particles having an antibody or an antigen bound thereto.

The invention also provides a reagent kit including the reagent.

In addition, the invention provides an automated immunoassay method including the step of mixing the reagent with a sample.

### Effects of the Invention

According to the present invention, microparticles having a reactive substance bound thereto can be stabilized in a dispersion liquid of the microparticles, for use in an immunoassay using an antigen-antibody reaction in the field of clinical examination or the like. According to the invention, a reagent containing microparticles having a reactive substance bound thereto whose reactivity is maintained over a long period of time can be provided. The reagent of the invention allows for the storage of the reagent over a long period of time without deterioration of measurement accuracy.

### Brief Description of Drawings

[Fig. 1] Fig. 1 is a graph showing the change over time in reactivity of a second reagent for cystatin C measurement (reagent of anti-cystatin C antibody-bound colloidal gold) where methionine is added in any of various concentrations.
[Fig. 2] Fig. 2 is a graph showing the change over time in reactivity of a second reagent for cystatin C measurement (reagent A of anti-cystatin C reduced IgG-bound colloidal gold) where any of a variety of suffur-containing compounds is added.
[Fig. 3] Fig. 3 is a graph showing the change over time in reactivity of a second reagent for cystatin C measurement (reagent B of anti-cystatin C reduced IgG-bound colloidal gold) where any of a variety of amino acids is added.

### Mode for Carrying Out the Invention

1. Method for stabilizing microparticles having a reactive substance bound thereto
The stabilization of microparticles having a reactive substance bound thereto ("reactive substance-bound microparticles") as used herein means that the reactivity of the microparticles having a reactive substance bound thereto does not substantially change over time, i.e., the reactivity is maintained over a long period of time. The stabilization of reactive substance-bound microparticles enables the provision of a reagent that contains such reactive substance-bound microparticles which can be stored over a long period of time without deterioration of measurement accuracy.

The method for stabilizing microparticles having a reactive substance bound thereto of the invention includes the step of allowing a sulfur-containing amino acid or a derivative thereof to coexist in a dispersion liquid of the microparticles.

The microparticles for binding a reactive substance thereto for use in the method of the invention are microparticles that can be used as an immunoassay reagent and preferably are latex particles and colloidal metal particles. Colloidal metal particles include colloidal particles of gold, silver, selenium, and the like, and it is generally easy to use colloidal gold particles and thus colloidal gold particles are preferably used. Commercially available colloidal gold particles may be used, or colloidal gold particles prepared according to a method commonly used by those skilled in the art, e.g., a method in which chloroauric acid is reduced by sodium citrate, may be used. The respective particle diameters of the colloidal gold particles are usually in the range of 5 to 100 nm and preferably in the range of 30 to 60 nm.

As for substances to be bound to the microparticles, any substances can be used as long as they specifically bind to an analyte. Examples thereof include substances that have a binding affinity including antibodies, reduced IgGs, antibody F(ab')₂ fragments or Fab' fragments, antigens, receptors, lectins, deoxyribonucleic acid (DNA), and ribonucleic acid (RNA).

In the method of the invention, a sulfur-containing amino acid or a derivative thereof is used. Herein, such compounds are called stabilizing substances. Stabilizing substances may be used singly or may be used as a combination of two or more.

The term "sulfizr-containing amino acid" as used herein refers to a naturally occurring sulfur-containing amino acid and a naturally occurring amino acid into which a sulfur atom is introduced. The term "derivative" conforms to the concept generally used in organic chemistry and refers to a substitution, an adduct, or the like.

As for the sulfur-containing amino acids for use as the stabilizing substance, examples of naturally occurring sulfur-containing amino acids include cysteine, cystine, and methionine, and examples of derivatives of sulfur-containing amino acids include methioninol, methioninal, methioninamide, 2-alkylcysteine, S-methylcysteine, cysteinol, cysteinal, and cysteinamide. Cysteine, cystine, and methionine are commercially available and preferably used in the invention. Methionine is particularly preferable.

According to the method of the invention, the stabilizing substance is allowed to coexist in a dispersion liquid of the reactive substance-bound microparticles. Means of allowing the stabilizing substance to coexist is not particularly limited. For example, the stabilizing substance may be added to a dispersion liquid of the reactive substance-bound microparticles; the reactive substance-bound microparticles may be added to the stabilizing substance or a dilution in which the stabilizing substance is diluted with a dispersion medium; or the reactive substance-bound microparticles and the stabilizing substance may be combined and the combination may be added to a dispersion medium. The dispersion medium is not particularly limited and, for example, water or a buffer fluid that uses a buffering agent and that will be described below may be used.

In the method of the invention, the concentration of the reactive substance-bound microparticles in the dispersion liquid may be such a concentration as is generally employed in the art.

In the method of the invention, the concentration of the stabilizing substance that coexists in the dispersion liquid is suitably selected depending on the type of the stabilizing substance. For example, in use of a sulfur-containing amino acid, the concentration is preferably 0.001 to 2.0 wt%, more preferably 0.005 to 1.0 wt%, and still more preferably 0.02 to 0.5 wt%.

Furthermore, in the method of the invention, a precipitation preventing substance may be suitably added to the dispersion liquid. The term "precipitation preventing substance" as used herein refers to a substance preventing the precipitation of the reactive substance-bound microparticles when the substance coexists in a dispersion liquid of the microparticles.

Examples of the precipitation preventing substance include polyanions and salts thereof, dextran, cyclodextrin, polyethylene glycol, and glycerol. Such precipitation preventing substances may be used singly or may be used as a combination of two or more.

As one type of the precipitation preventing substance, examples of polyanions include dextran sulfate, heparin, polystyrene sulfonate, hyaluronic acid, and chondroitin sulfate. Examples of polyanion salts include dextran sulfate sodium, heparin sodium, and the like. Among such polyanions and salts thereof, it is suitable to use a water-soluble polymeric compound having a sulfate group to attain a high precipitation preventing effect. Examples of such compounds include dextran sulfate, heparin, dextran sodium sulfate, and heparin sodium.

In the method of the invention, the concentration of the precipitation preventing substance coexisting in the dispersion liquid is suitably selected depending on the type of the precipitation preventing substance. For example, in use of a polyanion or a salt thereof, the concentration is preferably 0.3 to 5.0 wt%, more preferably 0.5 to 2.0 wt%, and still more preferably 0.7 to 1.1 wt%. In use of dextran or cyclodextrin, the concentration is preferably 0.1 to 5.0 wt%, more preferably 0.2 to 1.8 wt%, and still more preferably 0.5 to 1.5 wt%. In use of polyethylene glycol, the concentration is preferably 0.3 to 5.0 wt% and more preferably 0.5 to 3.0 wt%. In use of glycerol, the concentration is preferably 5 to 40 wt% and more preferably 10 to 35 wt%.

In the method of the invention, metal ions and chelating agents (as disclosed in Patent Document 2), at least one selected from the group consisting of calcium ions, magnesium ions, dextran sulfate, cholic acid, deoxycholic acid, xanthurenic acid, and salts thereof (as disclosed in Patent Document 3) may be further added to the dispersion liquid suitably as other stabilizing substances.

Furthermore, in the method of the invention, buffer, sugar, sugar alcohol, albumin, polyethylene glycol, sodium chloride, preservative, and other substances, which are conventionally used, may be suitably contained in the dispersion liquid.

According to the method of the invention, reactive substance-bound microparticles can be stabilized by allowing a stabilizing substance to coexist in a dispersion liquid of the reactive substance-bound microparticles. Accordingly, this method allows the reactivity of the microparticles to be maintained in the dispersion liquid over a long period of time. It is particularly useful when a precipitation preventing substance, such as a polyanion, dextran, or the like, coexist in the dispersion liquid.

2. Reagent which contains reactive substance-bound microparticles and stabilizing substance
The reagent of the invention contains the reactive substance-bound microparticles as described above and the stabilizing substance as described above, and contains, as needed, the precipitation preventing substance as described above, other stabilizing substances, buffer, sugar, sugar alcohol, albumin, sodium chloride, preservative, and other additives.

In the reagent of the invention, the reactive substance-bound microparticles are stabilized and therefore the reactivity of the microparticles are maintained over a long period of time. The temperature at which the reagent of the invention is stored is preferably 25°C or less, more preferably 10°C or less, and still more preferably 8°C or less. Preferably, the reagent of the invention is stored under a dark place and a low humidity.

The reagent of the invention is normally in a liquid form and used as a reagent that uses reactive substance-bound microparticles and especially as an immunoassay reagent commonly used in the art.

The concentration of the reactive substance-bound microparticles and the concentration of the stabilizing substance in the reagent of the invention are not particularly limited. For enhancing the stability of the reactive substance-bound microparticles, it is preferable that the concentrations are within the ranges adopted for the above-described method.

Examples of buffer that may be contained in the reagent of the invention include phosphate buffers; Tris-HCl buffers; succinate buffers; Good's buffers such as glycylglycine, MES (2-(N-morpholino)ethanesulfonic acid), HEPES (N-2-hydroxyethyl-piperadine-N'-ethanesulfonic acid), TES (N-tris(hydroxymethy)methyl-2-aminoethanesulfonic acid), MOPS (3-(N-morpholino)propanesulfonic acid), PIPES (piperazine-1,4-bis(2-ethanesulfonic acid)), and Bis-Tris (bis(2-hydroxyethyl)iminotris(hydroxymethyl)methane); and the like. It is preferable that the above mentioned buffer is contained such that the pH in the reagent is 5 to 9 or the concentration in the reagent is 1 to 100 mM.

Examples of sugar and sugar alcohol that may be contained in the reagent of the invention include glucose, mannose, saccharose, lactose, maltose, mannitol, sorbitol, and the like. The concentration of the sugar or sugar alcohol in the reagent is preferably 0.01 to 10.0 wt%.

Examples of albumin that may be contained in the reagent of the invention include bovine serum albumin (BSA) and the like. The concentration of the albumin in the reagent is preferably 0.001 to 1.0 wt%.

Examples of preservative that may be contained in the reagent of the invention include sodium azide and the like. The concentration of the preservative in the reagent is preferably 0.01 to 0.5 wt%.

Examples of other additives that may be contained in the reagent of the invention include Tween 20, polyethylene glycol lauryl ether, 5-bromosalicylic acid, sodium salicylate, sodium benzoate, sodium benzenesulfonate, phenol, thymol, and the like.

In the reagent of the invention, the reactive substance-bound microparticles are stabilized as described above and therefore the reactivity of the microparticles is maintained over a long period of time. Therefore, an immunoassay can be carried out with the reagent without deterioration of measurement accuracy even after the reagent being stored over a long period of time.

### 3. Reagent kit

The reagent kit of the invention includes a reagent which contains the reactive substance-bound microparticles and the stabilizing substance as described above (i.e., the reagent of the invention). The reagent kit is for use in, for example, an immunoassay, and especially an automated immunoassay, using latex agglutination, colloidal gold agglutination, or a like method.

In use of the reagent kit of the invention as an immunoassay reagent kit, the kit may be usually composed of a first reagent which contains components for the dilution and the pretreatment of the sample prior to the immunoreaction, and for assisting the immunoreaction, etc.; a second reagent of the reagent of the invention; and as needed, a reference standard of an analyte for preparing a calibration curve.

As an analyte in the reagent kit of the invention, various substances present in a sample may be selected depending on the reactive substance bound to the microparticles in the reagent of the invention. Examples of substances that may be measured (i.e., analytes) include proteins, such as albumin, hemoglobin, hemoglobin A1c, myoglobin, transferrin, lactoferrin, cystatin C, ferritin, α-fetoprotein, a carcinoembryonic antigen, CA 19-9, a prostatic-specific antigen, C-reactive protein (CRP), a fibrin degradation product (FDP), pepsinogens I and II, collagen, and the like; lipoproteins, such as high-density lipoprotein, low-density lipoprotein, very-low-density lipoprotein, and the like; nucleic acids, such as deoxyribonucleic acid, ribonucleic acid, and the like; enzymes, such as alkaline phosphatase, lactate dehydrogenase, lipase, amylase, and the like; immunoglobulins, such as IgG, IgM, IgA, IgD, IgE, and the like; antigens and antibodies associated with infectious diseases, such as hepatitis B virus, hepatitis C virus, human immunodeficiency virus, *Helicobacter pylori,* antibodies against these antigens, and the like; drugs, such as haloperidol, bromperidol, and the like; hormones, such as sex hormones and the like; and the like.

Examples of analyte-containing samples to be subjected to measurement include biological samples, such as blood, plasma, serum, urine, feces (suspension), spinal fluid, ascites, and the like; samples obtained from the environment or extracts thereof, and the like.

### 4. Automated immunoassay method

The automated immunoassay method of the invention is performed using the reagent of the invention. Since the reactive substance-bound microparticles are stabilized in the reagent and the reactivity is maintained over a long period of time, the automated immunoassay method can yield a highly accurate measured value even if the reagent is used after being stored over a long period of time.

Specifically, the automated immunoassay method of the invention is carried out as follows: a sample, a first reagent, and the reagent of the invention (second reagent) are set in an autoanalyzer and then the sample is automatically and successively mixed with the first reagent and the second reagent. The proportion of the first reagent to the second reagent may be suitably determined. The reagent of the invention (second reagent) is diluted preferably 2 to 9-fold and more preferably 3 to 5-fold.

### Examples

The present invention shall be described in more detail below by way of examples although the invention is not limited by the examples. In the examples below, the symbol "%" denotes weight/volume% (W/V%) unless specified otherwise.

### Preparation Example 1: Preparation of colloidal gold fluid

A 10% chloroauric acid aqueous solution (2 mL) was added to 1L of distilled water at 95°C while stirring, and then 10 mL of a 2% sodium citrate aqueous solution was added 1 minute later. The mixture was stirred for 20 minutes and then cooled to 30°C. After cooling, the pH was adjusted with a 0.1% potassium carbonate aqueous solution to be 7.1.

### Preparation Example 2: Preparation of anti-cystatin C antibody-bound colloidal gold reagent

An anti-cystatin C antibody (Dako Japan Inc.) was diluted to a concentration of 50 µg/mL with 10 mM HEPES (pH 7.1) containing 0.05% sodium azide. This anti-cystatin C antibody solution (100 mL) was added to about 1 L of the colloidal gold fluid prepared in Preparation Example 1 and the mixture was stirred for 2 hours under refrigeration. Moreover, 110 mL of 10 mM HEPES (pH 7.1) containing 5.46% mannitol, 0.5% bovine serum albumin (BSA), and 0.05% sodium azide was added to the mixture and they were stirred at 37°C for 90 minutes. Next, after the mixture was centrifuged at 8000 rpm for 40 minutes in order to remove the supernatant, about 1 L of 5 mM HEPES (pH 7.5) containing 3% mannitol, 0.1% BSA, and 0.05% sodium azide (solution A) was added to the residue to disperse the antibody-bound gold colloid. Furthermore, centrifugation was performed at 8000 rpm for 40 minutes in order to remove the supernatant therefrom, and the antibody-bound gold colloid was dispersed in solution A containing 1.5% dextran sodium sulfate so as to attain a total volume of 240 mL, thereby giving a reagent of anti-cystatin C antibody-bound colloidal gold.

### Preparation Example 3: Preparation of anti-cystatin C reduced IgG

An anti-cystatin C antibody (Dako Japan Inc.) was dialyzed with 10 mM HEPES (pH 7.1) containing 0.05% sodium azide, 5 mM EDTA·2Na, and 150 mM sodium chloride (solution B), and then diluted with solution B to a concentration of 10 mg/mL. To this antibody solution, 2-Mercaptoethylamine hydrochloride was added to a concentration of 6 mg/mL, and dissolved therein. Then, the solution was heated at 37°C for 90 minutes. With a gel filtration column, 2-Mercaptoethylamine hydrochloride was removed, thereby giving anti-cystatin C reduced IgG.

### Preparation Example 4: Preparation of anti-cystatin C reduced IgG-bound colloidal gold reagent

The anti-cystatin C reduced IgG prepared in Preparation Example 3 was diluted to a concentration of 50 µg/mL with 10 mM HEPES (pH 7.1) containing 0.05% sodium azide. This anti-cystatin C reduced IgG solution (100 mL) was added to about 1 L of the colloidal gold fluid prepared in Preparation Example 1 and the mixture was stirred for 2 hours under refrigeration. Moreover, 110 mL of 10 mM HEPES (pH 7.1) containing 5.46% mannitol, 0.5% BSA, and 0.05% sodium azide was added to the mixture and they were stirred at 37°C for 90 minutes. Next, after the mixture was centrifuged at 8000 rpm for 40 minutes in order to remove the supernatant, about 1 L of solution A was added to the residue to disperse the reduced IgG-bound gold colloid. Furthermore, centrifugation was performed at 8000 rpm for 40 minutes in order to remove the supernatant therefrom, and the antibody-sensitized gold colloid was dispersed in solution A containing 1.5% dextran sodium sulfate so as to attain a total volume of 240 mL, thereby giving a reagent A of anti-cystatin C reduced IgG-bound colloidal gold. The antibody-sensitized gold colloid was dispersed in solution A containing 1.25% dextran sodium sulfate so as to attain a total volume of 240 mL, thereby giving a reagent B of anti-cystatin C reduced IgG-bound colloidal gold.

### Preparation Example 5: Preparation of a first reagent for cystatin C measurement

To 0.5 M Bis-Tris (pH 6.7) buffer containing 5% sodium chloride, 0.2% EDTA, 0.2% sodium alkylphenyldisulfonate, and 0.35% polyoxyethylene lauryl ether, polyethylene glycol was added as a reaction accelerator in a proportion of about 1.0 to 2.5%, thus giving a first reagent for cystatin C measurement.

### Example 1: Examination for addition effect of methionine

To the reagent of anti-cystatin C antibody-bound colloidal gold prepared in Preparation Example 2, methionine was added to a concentration of 0.005%, 0.02%, 0.05%, 0.1%, or 0.5% to give a second reagent for cystatin C measurement. In the measurement of a sample where cystatin C is contained at a concentration of 8 mg/L, the reactivity of the second reagent was examined for the change over time due to storage of the reagent. The anti-cystatin C antibody-bound colloidal gold reagent where no methionine was added was regarded as a control of the second reagent for cystatin C measurement. The second reagent was stored at 25°C, and was examined at the beginning, 4 days later, 8 days later, 20 days later, and 39 days later using the first reagent prepared in Preparation Example 5 according to the procedure described below.

### (The procedure of Examination)

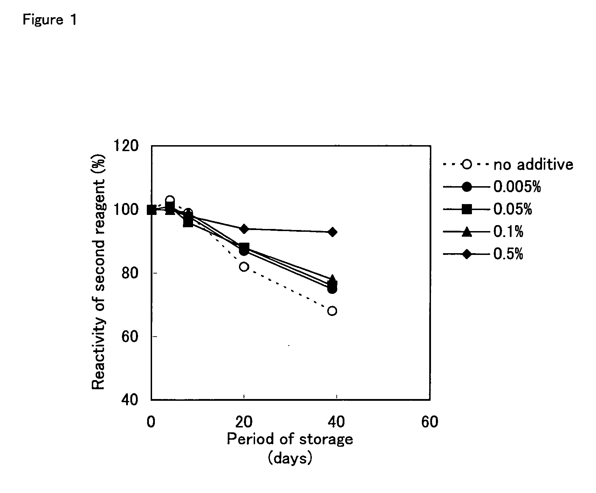

Into 3 µL of the sample, 200 µL of a first reagent was poured and the mixture was heated at 37°C for about 5 minutes. Next, 100 µL of a second reagent was poured to the mixture, which was then reacted at 37°C. The absorbance change at the photometric points from 18 to 24 was measured with Hitachi 7070 autoanalyzer at a dominant wavelength of 505 nm and a complementary wavelength of 660 nm. The proportion of the absorbance change after a period of storage relative to the absorbance change at the beginning of storage was expressed as the reactivity (%) of the second reagent. The results are shown in Table 1 and Figure 1.

**[Table 1]**

| Concentration of methionine (%) | Reactivity of second reagent* (Absorbance change) | | | | |
|---|---|---|---|---|---|
| | Beginning | 4 days later | 8 days later | 20 days later | 39 days later |
| 0 | 100% | 103% | 99% | 82% | 68% |
| | (0.6743) | (0.6931) | (0.6681) | (0.5510) | (0.4601) |
| 0.005 | 100% | 101% | 98% | 87% | 75% |
| | (0.6706) | (0.6781) | (0.6546) | (0.5847) | (0.5037) |
| 0.02 | 100% | 99% | 97% | 86% | 74% |
| | (0.6795) | (0.6710) | (0.6581) | (0.5816) | (0.5055) |
| 0.05 | 100% | 101% | 96% | 88% | 76% |
| | (0.6861) | (0.6909) | (0.6607) | (0.6007) | (0.5200) |
| 0.1 | 100% | 100% | 99% | 88% | 78% |
| | (0.7073) | (0.7080) | (0.6975) | (0.6251) | (0.5491) |
| 0.5 | 100% | 100% | 98% | 94% | 93% |
| | (0.6788) | (0.6770) | (0.6660) | (0.6362) | (0.6346) |

| | | | | | |
|---|---|---|---|---|---|
| *: The proportion of each absorbance change when the absorbance change at the beginning of storage is considered as 100%. | | | | | |

As is clear from the results presented in Table 1 and Figure 1, at 20 days later of storage, while a decrease of the reactivity by about 20% was observed for the second reagent where methionine was not added, the reactivity was decreased by about 5% for the second reagent where methionine was contained in a proportion of 0.5%. Also, at 39 days later of storage, while a decrease of the reactivity by 30% or greater was observed for the second reagent where methionine was not added, the reactivity was decreased by about 7% for the second reagent where methionine was contained in a proportion of 0.5%. The reactivity at 39 days later of storage for the second reagent where methionine was contained even in a proportion of 0.005% was about 7% higher than that for the second reagent where methionine was not added. Therefore, there was the effect of stabilization observed due to the addition of methionine. Accordingly, it was found that antibody-bound colloidal gold particles could be stabilized by allowing methionine to coexist in a dispersion liquid of the particles.

### Example 2: Examination for addition effect of methionine and cysteine

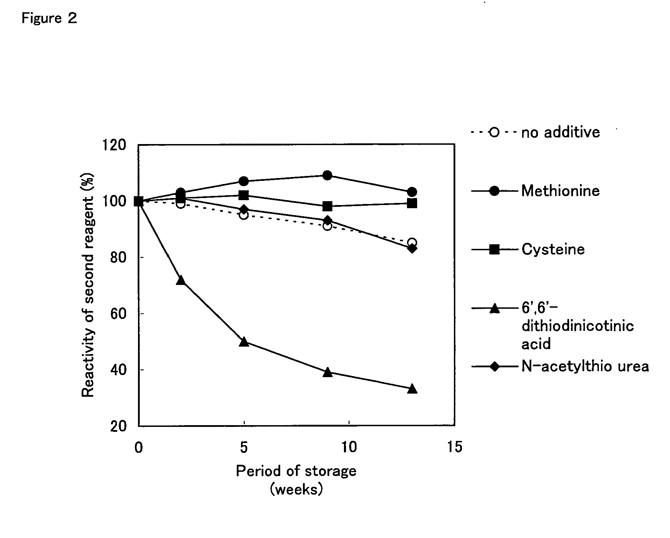

To the reagent A of anti-cystatin C reduced IgG-bound colloidal gold prepared in Preparation Example 4, methionine or cysteine was added at a concentration for methionine of 0.03%, 0.06% or 0.12%, or a concentration for cysteine of 0.03%, to give a second reagent for cystatin C measurement. In the measurement of a sample where cystatin C is contained at a concentration of 8 mg/L, the second reagent was examined for the change over time in reactivity due to storage of the reagent. The anti-cystatin C reduced IgG-bound colloidal gold reagent A where 6',6'-dithiodinicotinic acid or N-acetylthio urea was added to a concentration of 0.03%, and the anti-cystain C reduced IgG-bound colloidal gold reagent A where no additive was added were regarded as controls of the second reagent for cystatin C measurement. The second reagent was stored under refrigeration and then subjected to the examination at the beginning, 2 weeks later, 5 weeks later, 9 weeks later, and 13 weeks later in the same manner as in Example 1 using the first reagent prepared in Preparation Example 5. The results are show in Table 2 and Figure 2.

**[Table 2]**

| Sulfur-containing compounds | Concentration (%) | Reactivity of second reagent* (Absorbance change) | | | | |
|---|---|---|---|---|---|---|
| | | Beginning | 2 weeks later | 5 weeks later | 9 weeks later | 13 weeks later |
| - | - | 100% | 99% | 95% | 91% | 85% |
| | | (0.9887) | (0.9766) | (0.9395) | (0.8964) | (0.8382) |
| Methionine | 0.03 | 100% | 103% | 107% | 109% | 103% |
| | | (0.9764) | (1.0068) | (1.0425) | (1.0626) | (1.0051) |
| | 0.06 | 100% | 102% | 106% | 109% | 106% |
| | | (0.9857) | (1.0016) | (1.0426) | (1.0753) | (1.0447) |
| | 0.12 | 100% | 103% | 107% | 109% | 107% |
| | | (0.9759) | (1.0097) | (1.0433) | (1.0665) | (1.0396) |
| Cysteine | 0.03 | 100% | 101% | 102% | 98% | 99% |
| | | (0.9066) | (0.9115) | (0.9210) | (0.8897) | (0.8992) |
| 6',6'-dithiodinicotinic acid | 0.03 | 100% | 72% | 50% | 39% | 33% |
| | | (0.6734) | (0.4847) | (0.3378) | (0.2632) | (0.2193) |
| N-acetylthio urea | 0.03 | 100% | 101 % | 97% | 93% | 83% |
| | | (0.9626) | (0.9688) | (0.9384) | (0.8984) | (0.8031) |

| | | | | | | |
|---|---|---|---|---|---|---|
| *: The proportion of each absorbance change when the absorbance change at the beginning of storage is considered as 100%. | | | | | | |

As is clear from the results presented in Table 2 and Figure 2, at 13 weeks later of storage, while a decrease of the reactivity by about 15% was observed for the second reagent where no additive was added, a decrease was barely observed for the second reagent where methionine or cysteine was contained. On the other hand, a decrease observed for the second reagent where 6',6'-dithiodinicotinic acid or N-acetylthio urea, either of which was sulfur-containing compounds but was not amino acids or derivatives thereof, was contained was equivalent to or greater than that observed for the second reagent where no additive was added. Accordingly, it was found that reduced IgG-bound colloidal gold particles could be stabilized by allowing methionine or cysteine to coexist in a dispersion liquid of the particles.

### Example 3: Examination for addition effect of amino acids

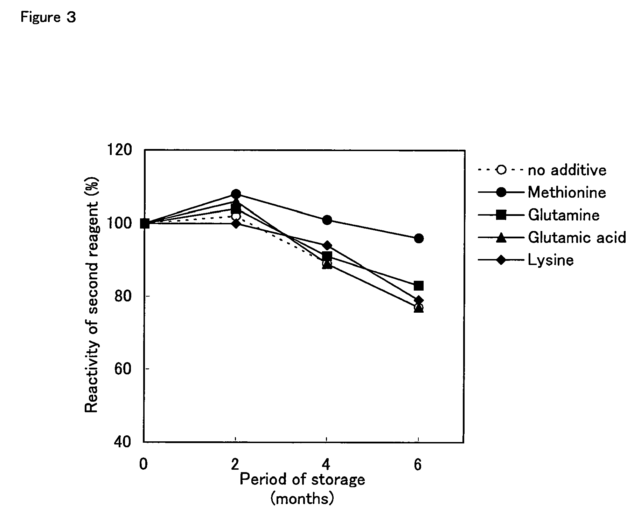

To the reagent B of anti-cystatin C reduced IgG-bound colloidal gold prepared in Preparation Example 4, methionine, glutamine, glutamic acid, or lysine was added to attain a concentration of 0.02% to give a second reagent for cystatin C measurement. In the measurement of a sample where cystatin C is contained at a concentration of 4 mg/L, the second reagent was examined for the change over time in reactivity due to storage of the reagent. The anti-cystatin C reduced IgG-bound colloidal gold reagent B where no additive amino acid was added regarded as a control of the second reagent for cystatin C measurement. The second reagent was stored under refrigeration and then subjected to the examination in the same manner as in Example 1 using the first reagent prepared in Preparation Example 5 at the beginning, 2 months later, 4 months later, and 6 months later. The results are shown in Table 3 and Figure 3.

**[Table 3]**

| Amino acid | | Reactivity of second reagent* (Absorbance change) | | | |
|---|---|---|---|---|---|
| | | Beginning | 2 months later | 4 months later | 6 months later |
| | | 100% | 102% | 89% | 77% |
| - | | (0.4524) | (0.4594) | (0.4046) | (0.3490) |
| | Agglomerate generation | - | + | 2+ | 2+ |
| | | 100% | 108% | 101% | 96% |
| Methionine | | (0.4393) | (0.4724) | (0.4442) | (0.4213) |
| | Agglomerate generation | - | - | - | - |
| | | 100% | 104% | 91% | 83% |
| Glutamine | | (0.4355) | (0.4533) | (0.3965) | (0.3636) |
| | Agglomerate generation | - | + | 2 + | 2+ |
| | | 100% | 106% | 89% | 77% |
| Glutamic acid | | (0.4335) | (0.4605) | (0.3871) | (0.3329) |
| | Agglomerate generation | - | + | 2+ | 2+ |
| | | 100% | 100% | 94% | 79% |
| Lysine | | (0.4418) | (0.4438) | (0.4143) | (0.3490) |
| | Agglomerate generation | - | + | 2+ | 2+ |

| | | | | | |
|---|---|---|---|---|---|
| *: The proportion of each absorbance change when the absorbance change at the beginning of storage is considered as 100°6. -: no agglomerate, +: some agglomerate, 2+: lots of agglomerate | | | | | |

As is clear from the results presented in Table 3 and Figure 3, for the second reagent where glutamine, glutamic acid, or lysine was contained as the amino acid, the reactivity was observed to be decreased as much as observed for the second reagent where no additive amino acid was added. However, for the second reagent where methionine was contained, a decrease was barely observed even at 6 months later. Also, agglomerate generation due to autoagglutination was not observed for the second reagent where methionine was contained. Accordingly, it was found that reduced IgG-bound colloidal gold particles could be stabilized by allowing, among other amino acids, a sulfur-containing amino acid, methionine, to coexist in a dispersion liquid of the particles.

### Industrial Applicability

According to the invention, reactive substance-bound microparticles can be stabilized. Therefore, it is possible to provide a reagent in which such microparticles are stabilized. In the reagent, the reactivity of the microparticles is maintained over a long period of time. Therefore, an immunoassay can be carried out with the reagent without deterioration of measurement accuracy even after the reagent being stored over a long period of time. The reagent and a reagent kit including such a reagent are particularly useful for an automated immunoanalyzer.

## Claims

1. A method for stabilizing microparticles having a reactive substance bound thereto, comprising:
allowing a sulfur-containing amino acid or a derivative thereof to coexist in a dispersion liquid of the microparticles.

2. A method according to claim 1, wherein the sulfur-containing amino acid is at least one selected from the group consisting of cysteine, cystine, and methionine.

3. A method according to claim 1 or 2, wherein the sulfur-containing amino acid or the derivative thereof is contained in the dispersion liquid in a proportion of 0.001 to 2.0 weight %.

4. A method according to any one of claims 1 to 3, comprising allowing a precipitation preventing substance to further coexist in the dispersion liquid.

5. A method according to claim 4, wherein the precipitation preventing substance is at least one selected from the group consisting of polyanions and salts thereof, dextran, cyclodextrin, polyethylene glycol, and glycerol.

6. A method according to claim 5, wherein the polyanion is dextran sulfate or heparin.

7. A method according to any one of claims 1 to 6, wherein the microparticles having a reactive substance bound thereto are colloidal gold particles having an antibody or an antigen bound thereto.

8. A reagent comprising microparticles having a reactive substance bound thereto and a sulfur-containing amino acid or a derivative thereof

9. A reagent according to claim 8, wherein the sulfur-containing amino acid is at least one selected from the group consisting of cysteine, cystine, and methionine.

10. A reagent according to claim 8 or 9, wherein the sulfur-containing amino acid or the derivative thereof is contained in the reagent in a proportion of 0.001 to 2.0 weight %.

11. A reagent according to any one of claims 8 to 10, further comprising a precipitation preventing substance.

12. A reagent according to claim 11, wherein the precipitation preventing substance is at least one selected from the group consisting of polyanions and salts thereof, dextran, cyclodextrin, polyethylene glycol, and glycerol.

13. A reagent according to claim 12, wherein the polyanion is dextran sulfate or heparin.

14. A reagent according to any one of claims 8 to 13, wherein the microparticles having a reactive substance bound thereto are colloidal gold particles having an antibody or an antigen bound thereto.

15. A reagent kit comprising the reagent of any one of claims 8 to 14.

16. An automated immunoassay method comprising mixing the reagent of any one of claims 8 to 14 with a sample.
